# EUROPÄISCHE PATENTSCHRIFT

(11) **EP 1 280 831 B1**
(45) Veröffentlichungstag und Bekanntmachung des Hinweises auf die Patenterteilung: **02.08.2006**
(21) Anmeldenummer: 01956434.3
(22) Anmeldetag: 11.05.2001
(51) Int. Cl.: C08F 8/18

(54) **VERFAHREN ZUR HERSTELLUNG VON POLYMERGEBUNDENEM 2-CHLORTRITYLCHLORID**
METHOD FOR PRODUCING POLYMER-BONDED 2-CHLOROTRITYL CHLORIDE
PROCEDE DE PRODUCTION DE 2-CHLORTRITYLCHLORURE LIE A UN POLYMERE

(30) Priorität: 12.05.2000 EP 00110039
(43) Veröffentlichungstag der Anmeldung: 05.02.2003
(73) Patentinhaber: Lonza AG, 4052 Basel (CH)
(72) Erfinder: MEININGHAUS, Carsten, CH-3930 Visp (CH)
(86) Internationale Anmeldenummer: PCT/EP2001/005416
(87) Internationale Veröffentlichungsnummer: WO 2001/085758

(56) Entgegenhaltungen:
- WO-A-92/22591
- US-A- 4 346 187
- PATENT ABSTRACTS OF JAPAN vol. 199, no. 612, 26. Dezember 1996 (1996-12-26) & JP 08 217794 A (BIO KOSUMOSU K.K.), 27. August 1996 (1996-08-27)
- PATENT ABSTRACTS OF JAPAN vol. 011, no. 246 (C-439), 11. August 1987 (1987-08-11) & JP 62 054703 A (KANEGAFUCHI CHEM. IND. CO., LTD.), 10. März 1987 (1987-03-10)

## Beschreibung

Die Erfindung betrifft ein Verfahren zur Herstellung von polymergebundenem 2-Chlortritylchlorid der Formel 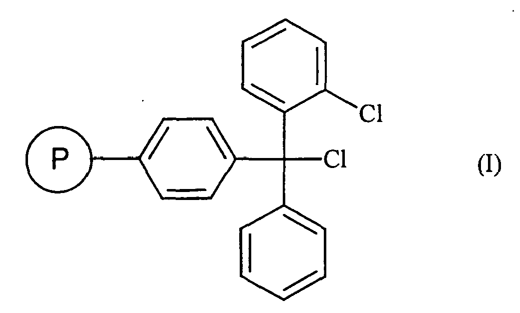 worin das Symbol Ⓟ einen polymeren Träger und vorzugsweise ein vemetztes Polystyrolharz bedeutet.

Polymergebundenes 2-Chlortritylchlorid ist ein kommerziell erhältliches (Calbiochem-Novabiochem AG, Läufelfingen, Schweiz, Produkt Nr. 01-64-0021) Reagenz zur Festphasensynthese von Peptiden. Es wird hierbei zunächst mit einer N-geschützten Aminosäure, die den C-Terminus des zu synthetisierenden Peptids bildet, zu dem entsprechenden Tritylester umgesetzt. Nach Aufbau der Peptidkette wird das Peptid durch Behandlung mit einer Carbonsäure, beispielsweise verdünnter Trifluoressigsäure oder Essigsäure, abgespalten, wobei sich der entsprechende polymergebundene Tritylester der Formel 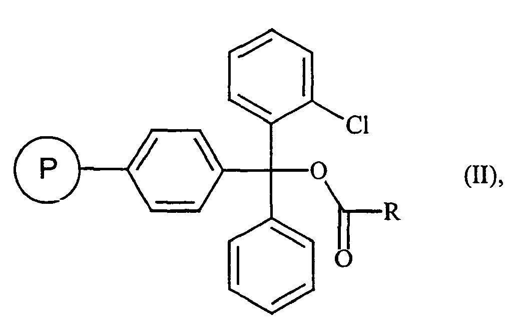 worin R eine C₁₋₄-Alkyl- oder C₁₋₄-Haloalkylgruppe und vorzugsweise Methyl oder Trifluormethyl bedeutet, bildet. Das Harz ist damit verbraucht und kann in dieser Form nicht mehr zu weiteren Synthesen eingesetzt werden.

Unter C₁₋₄-Alkyl sind hier und im Folgenden alle Alkylgruppen mit 1 bis 4 Kohlenstoffatomen zu verstehen, also Methyl, Ethyl, Propyl, Isopropyl, Butyl, Isobutyl, *sec*-Butyl und tert-Butyl. Unter C₁₋₄-Haloalkyl sind entsprechend alle C₁₋₄-Alkylgruppen mit einem oder mehreren gleichen oder verschiedenen Halogenatomen als Substituenten zu verstehen, vorzugsweise perfluorierte C₁₋₄-Alkylgruppen wie Trifluormethyl.

Aufgabe der vorliegenden Erfindung war daher, ein Verfahren bereitzustellen, das aus dem nach Abspaltung des Peptids anfallenden acyloxylierten Harz (II) wieder das polymergebundene 2-Chlortritylchlorid regeneriert, welches dann erneut zu Peptidsynthesen verwendet werden kann.

Erfindungsgemäss wird diese Aufgabe durch das Verfahren nach Patentanspruch 1 gelöst.

Es wurde gefunden, dass durch einfaches Behandeln des acyloxylierten Harzes (II) mit Chlorwasserstoff in einem organischen Lösungsmittel das polymergebundene 2-Chlortritylchlorid (I) regeneriert werden kann. Da es sich bei dem Austausch von Carboxylat gegen Chlorid offenbar um eine Gleichgewichtsreaktion handelt, wird die Behandlung vorteilhaft mehrmals mit frischem Lösungsmittel wiederholt, um die freigesetzte Carbonsäure aus dem System zu entfernen und so einen vollständigen Austausch zu erzielen. Es ist auch möglich, eine Packung des acyloxylierten Harzes (II) mit einer Lösung von Chlorwasserstoff zu perkolieren, bis das Carboxylat vollständig gegen Chlorid ausgetauscht ist.

Als Lösungsmittel eignet sich grundsätzlich jedes wasserfreie organische Lösungsmittel, das nicht mit Chlorwasserstoff reagiert und für diesen ein hinreichendes Lösevermögen besitzt. Vorzugsweise wird Dichlormethan als Lösungsmittel eingesetzt.

Das erfindungsgemässe Verfahren kann sowohl mit einer vorbereiteten Lösung von Chlorwasserstoff als auch mit einer durch Einleiten von gasförmigem Chlorwasserstoff in einen mit dem Lösungsmittel und dem acyloxylierten Harz (II) beschickten Reaktor in situ erzeugten Lösung durchgeführt werden.

Das folgende Beispiel verdeutlicht die Durchführung des erfindungsgemässen Verfahrens, ohne dass darin eine Einschränkung zu sehen ist.

### Beispiel

In einen Doppelmantelreaktor zur Festphasen-Peptidsynthese (zylindrisches Glasgefäss mit Temperiermantel und Glasfritte im unteren Teil) wurden 5 g trifluoracetoxyliertes Harz (II) gegeben und mit 20 ml Dichlormethan versetzt. Durch die Fritte wurde zunächst für 10 min Stickstoff geleitet und die Anordnung auf 5 °C gekühlt. Dann wurde durch die Fritte ein schwacher Chlorwasserstoffstrom geleitet, wobei der aus dem Reaktor entweichende überschüssige Chlorwasserstoff in Waschflaschen mit Natronlauge absorbiert wurde. Nach ca. 10 min wurde die Lösung abgesaugt und durch frisches Dichlormethan ersetzt. Anschliessend wurde wiederum für ca. 10 min Chlorwasserstoff eingeleitet. Dieser Vorgang wurde insgesamt fünfmal durchgeführt. Danach wurde das Harz unter Stickstoff abgesaugt und über Nacht bei 30 °C getrocknet. Das so erhaltene polymergebundene 2-Chlortritylchlorid wies in der Bindungskapazität für Aminosäuren keine signifikanten Unterschiede zu dem kommerziell erhältlichen Produkt auf.

## Patentansprüche

1. Verfahren zur Herstellung von polymergebundenem 2-Chlortritylchlorid der Formel 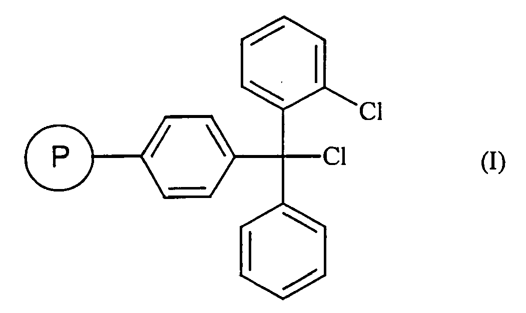 worin Ⓟ einen polymeren Träger, vorzugsweise vemetztes Polystyrol, bedeutet, **dadurch gekennzeichnet, dass** der entsprechende Carbonsäureester der Formel 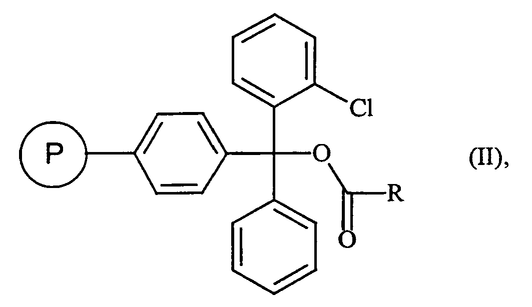 worin R eine C₁₋₄-Alkyl- oder C₁₋₄-Haloalkylgruppe bedeutet, mit einer Lösung von Chlorwasserstoff in einem organischen Lösungsmittel behandelt wird.

2. Verfahren nach Anspruch 1, **dadurch gekennzeichnet, dass** R Methyl oder Trifluormethyl ist.

3. Verfahren nach Anspruch 1 oder 2, **dadurch gekennzeichnet, dass** als organisches Lösungsmittel Dichlormethan verwendet wird.

4. Verfahren einem der Ansprüch 1 bis 3, **dadurch gekennzeichnet, dass** die Behandlung mehrmals mit frischer Lösung wiederholt wird.

5. Verfahren nach einem der Ansprüche 1 bis 4, **dadurch gekennzeichnet, dass** die Lösung von Chlorwasserstoff im organischen Lösungsmittel in situ durch Einleiten von Chlorwasserstoffgas hergestellt wird.

6. Verfahren nach einem der Ansprüche 1 bis 3, **dadurch gekennzeichnet, dass** eine Packung des polymergebundenen Carbonsäuretritylesters (II) mit der Lösung von Chlorwasserstoff im organischen Lösungsmittel perkoliert wird.

## Claims

1. Process for the preparation of polymer-bound 2-chlorotrityl chloride of the formula 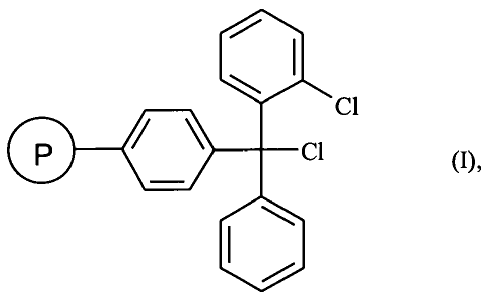 in which Ⓟ is a polymeric support, preferably crosslinked polystyrene, **characterized in that** the corresponding carboxylic acid ester of the formula 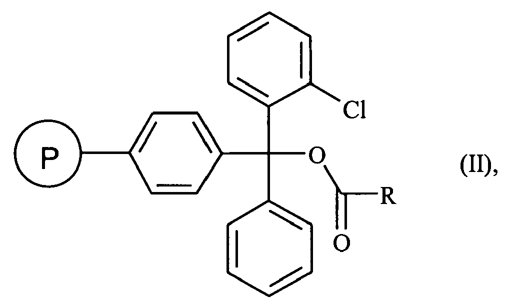 in which R is a C₁₋₄-alkyl or C₁₋₄-haloalkyl group, is treated with a solution of hydrogen chloride in an organic solvent.

2. Process according to Claim 1, **characterized in that** R is methyl or trifluoromethyl.

3. Process according to Claim 1 or 2, **characterized in that** the organic solvent used is dichloromethane.

4. Process according to Claims 1 to 3, **characterized in that** the treatment is repeated a number of times with fresh solution.

5. Process according to one of claims 1 to 4, **characterized in that** the solution of hydrogen chloride in the organic solvent is prepared *in situ* by passing in hydrogen chloride gas.

6. Process according to one of Claims 1 to 3, **characterized in that** a packing of the polymer-bound carboxylic acid trityl ester (II) is percolated with the solution of hydrogen chloride in the organic solvent.

## Revendications

1. Procédé de préparation de chlorure de 2-chlorotrityle lié à un polymère, de formule 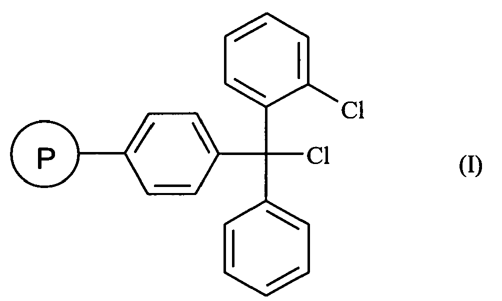 dans laquelle Ⓟ est un support polymère, de préférence un polystyrène réticulé, **caractérisé en ce que** l'ester d'acide carboxylique correspondant de formule 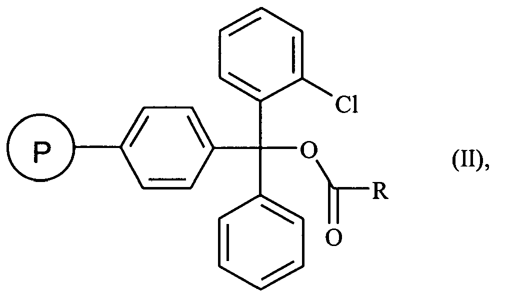 dans laquelle R représente un groupe alkyle en C₁₋₄ ou un groupe halogénoalkyle en C₁₋₄, est traité avec une solution de chlorure d'hydrogène dans un solvant organique.

2. Procédé selon la revendication 1, **caractérisé en ce que** R représente un groupe méthyle ou un groupe trifluorométhyle.

3. Procédé selon la revendication 1 ou 2, **caractérisé en ce que** le solvant organique utilisé est le dichlorométhane.

4. Procédé selon une des revendications 1 à 3, **caractérisé en ce que** le traitement este répété plusieurs fois avec de la solution fraîche.

5. Procédé selon une des revendications 1 à 4, **caractérisé en ce que** la solution de chlorure d'hydrogène dans le solvant organique est préparé in situ par introduction de chlorure d'hydrogène gazeux.

6. Procédé selon une des revendications 1 à 3, **caractérisé en ce qu'**un garnissage de l'ester tritylique d'acide carboxylique (II) lié à un polymère est percolé avec la solution de chlorure d'hydrogène dans le solvant organique.
